# EUROPEAN PATENT SPECIFICATION

(11) **EP 4 415 666 B1**
(45) Date of publication and mention of the grant of the patent: **09.12.2026**
(21) Application number: 22800840.5
(22) Date of filing: 13.10.2022
(51) Int. Cl.: A61F 9/008

(54) **DUAL 532 AND 577 NM LASER DELIVERY SYSTEM ATTACHED TO AN OPHTHALMIC MICROSCOPE**
DOPPEL 532 UND 577 NM LASERABGABESYSTEM BEFESTIGT AN EINEM OPHTHALMISCHEN MIKROSKOP
SYSTÈME D'ÉMISSION LASER DOUBLE 532 ET 577 NM FIXÉ À UN MICROSCOPE OPHTALMIQUE

(30) Priority: 14.10.2021 IN 202141031550
(43) Date of publication of application: 21.08.2024
(73) Proprietor: Appasamy Associates Pvt Ltd, Chennai 600106 (IN)
(72) Inventor: RAVILLA KASTHURI, Narayanasamy, Chennai, 600106 (IN); JEYARAJ, Sri Rama Prasad, Chennai, 600106 (IN)
(74) Representative: Dall'Olio, Christian
(86) International application number: PCT/IN2022/050912
(87) International publication number: WO 2023/062650

(56) References cited:
- US-A- 5 954 711
- US-A1- 2013 072 917
- US-A1- 2014 107 634
- US-B2- 10 433 718

## Description

### FIELD OF INVENTION

The present invention relates to a laser unit, particularly to dual (532 and 577) nm laser delivery system comprising to an existing ophthalmic operating microscope for creation of a Capsulorhexis in a human eye .

### BACKGROUND OF INVENTION

Capsulorhexis generally refer to removal of a part of the capsule by shear and stretch forces, but in situations like a developmental cataract a part of the capsule is also removed by a similar technique. A cataract is a dense, cloudy area that forms in the lens of an eye. Creation of the Capsulorhexis is a key stage in cataract surgery, and producing a precise rhexis is of paramount importance. Capsulorhexis was first performed by removing the lens and safely implanting intraocular lens (IOL) in the capsular bag. This procedure avoided the radial tears which occurred frequently in the can-opener capsulotomies that were performed at the time. A correctly sized, and a well-centered Capsulorhexis with a 360° overlapping capsular edge prevents IOL tilt, myopic shift, optic de-centration, and posterior and anterior capsular opacification, partly due to a combination of the symmetric contractile forces of the capsular bag, and the shrink wrap effect. However, eccentric or irregularly shaped Capsulorhexis with a diameter extending beyond the optic edge may lose these advantages.

Currently, Capsulorherxis step is being done during Phacosurgery either by manually using a 26G needle or forceps. The selection depends on the length of the incision that the surgeon chooses to make. However, the manual procedure is time consuming and also involves surgeon's skill. Also, in manual procedure, it is essential that capsule tear direction is controlled, as the overall direction of pulling forces on the capsule flap can cause different tearing direction patterns. Recently Capsulorherxis step is being done using a Femtosecond laser which is a newer technology and very expensive investment too for individual practitioners. Further, it also requires a separate specialized operation room with accurately maintained room temperature for its perfect functioning hence very high recurring maintenance cost. However, in the Femtosecond laser assisted cataract surgery, the laser equipment and the ultrasonic emulsification equipment are not integrated, the operation process needs to be carried out separately in two different operation rooms, which also involves shifting of the patient from one operating room to an another operating room and hence the operation time is prolonged. In addition, patients with primary diseases of small pupils and corneal epithelium and adverse eye socket shapes to the connection of laser equipment and eyes are not suitable for Femtosecond laser-assisted cataract surgery.

US11083625B2 discloses a system for laser ophthalmic surgery comprising of laser assembly, confocal detection assembly and scanning assembly. The system has used two treatment lasers. First treatment laser is infrared wavelength 870 nm to 1200 nm and second treatment laser is ultraviolet wavelength 320 nm to 370 nm. The focal spot size is 150 microns.

US9895056B2 discloses an ophthalmic laser illuminator with three different wavelength lasers Red (At least 620 nm), Blue (less than 480 nm), Green (between 552 nm and 557 nm). The illuminator has mixer/homogenizer for mixing the three wavelengths of light.

US8315280B2 relates to multiple wavelength laser system which includes at least two semiconductor diode lasers and one light guide system. The wavelength ranges are 480 nm to 520 nm, 530 to 550 nm, 560 to 580 nm and 1040 nm to 1070 nm. However, the purpose is for therapeutic sectors that include photocoagulation, transpupillary thermotherapy, photothermal tumor treatment and photodynamic therapy.

US5426662A discloses a laser system for ophthalmological treatments with two competing wavelength regions. The wavelength regions are reflective at 647 nm and transmissive at 531 nm and 568 nm.

US10022270B2 discloses methods and systems for performing laser-assisted surgery. The wavelength range is from 500 nm to 1100 nm and the focal spot has a diameter of 1 to 20 microns.

US10596037B2 relates to an ophthalmic phototherapy device which promotes the healing of damaged or diseased eye tissue. The system uses multiple wavelengths and the ranges are 500 nm to 650 nm or 640 nm to 700 nm or 800 nm to 900 nm.

WO2015131135A1discloses laser assisted cataract surgery methods and devices which utilize one or more treatment laser beams to create a shaped opening in the anterior lens capsule of the eye when performing a capsulorrhexis procedure. The laser may operate at a wavelength of about 577 nm or about 590 nm or about 810 nm. The spot size of laser is 100 microns to 350 microns.

KR102190881B1 discloses a laser device for high-intensity pain treatment using a composite wavelength and a programmed scan handpiece. The wavelength band of the laser beam is any one of 980 nm and 1064 nm in the case of a short wavelength, and provides a combined wavelength of 980 nm and 1064 nm in the case of a complex wavelength.

WO1993001638A1 discloses a laser system with dielectric mirror, laser medium and motor driven carriage. The laser medium utilizing a YAG type crystal to make it possible, the generation of wavelengths of 1064 nm, 1320 nm, 532 nm and 660nm from a single laser system. KR101177478B1 discloses a laser apparatus with laser oscillating unit, power converting unit, an optical coupling unit, laser output cut-off unit and a scope unit to controls the direction or spot size of a laser beam. The laser apparatus use dual wavelength namely yellow wavelength (578 nm) and green wavelength (511 nm).

Accordingly, there exists a need for a compact, precision, safety and cost effective dual wavelength laser unit with a delivery system that comprises an existing ophthalmic operating microscope to overcome the afore-mentioned drawbacks.

### OBJECTS OF INVENTION

One or more of the problems of the conventional prior art may be overcome by various embodiments of the system of the present invention.

It is the primary object of the present invention to provide a compact, cost effective dual wavelength laser unit with a delivery system comprising ophthalmic operating microscope .

It is another object of the present invention to provide a dual wavelength laser delivery system comprising an existing ophthalmic operating microscope to create a circular pattern to accomplish a pre-measured diameter size of Capsulorhexis with a precise circular cut of the capsule in human eye with a precise diameter of 3 mm to 6 mm in steps of 0.5 mm.

It is another object of the present invention to provide a Galvo assembly driven dual wavelength laser delivery system comprising an existing ophthalmic operating microscope for laser beam to be focused on to the capsular bag in the human eye.

It is another object of the present invention, wherein the laser delivery system converges and focuses the dual wavelength laser beam to create a circular pattern in order to accomplish a pre-measured Capsulorhexis with a precise circular cut of the capsule in the human eye with a precise diameter of 3 mm to 6 mm in steps of 0.5 mm.

It is another object of the present invention, wherein the laser delivery system has an aiming beam which is used to locate and guide user to aim the dual wavelength laser beam before the Capsulorhexis.

### SUMMARY OF INVENTION

Thus according to the basic aspect of the present invention, there is provided a dual wavelength laser delivery system comprising an existing ophthalmic operating Said system comprises of:
a dual wavelength laser unit ;
a delivery system; and
an ophthalmic operating microscope,
wherein the dual wavelength laser unit comprises of a green laser module 532 nm and a yellow laser module 577 nm along with an aiming beam 635 nm within both the laser modules to create a circular pattern with a precise circular cut of the capsule in a human eye,
wherein the aiming beam 635 nm is used to locate and guide user to aim the 535 nm and/or 577 nm laser beam,
wherein each of the laser modules comprises of a low pass filter and a focusing lens respectively,
wherein the green and yellow laser modules are connected to the delivery system through an interchangeable single fiber optic cable,
wherein the delivery system comprises of a right-angle prism; a X and Y Galvo assembly a focusing lens assembly, and a folding mirror
wherein the laser beams generated in the green laser module 532 nm (3) and yellow laser module 577 nm (4) from the laser unit (1) pass through the fiber optic cable (6) gets reflected using the right angle prism, gets deflected by the X and Y Galvo assembly, enters the focusing lens assembly and gets reflected by a folding mirror and focused on the human eye capsule at 90 degrees, and
wherein said laser delivery system comprising the operating microscope's converges and focuses the laser wavelength of 532 nm and/or 577 nm laser beam in-line with the 635 nm aiming beam to create a circular pattern to accomplish a pre-measured Capsulorhexis with a precise circular cut of the capsule in the human eye.

It is another aspect of the present invention, wherein the low pass filters are mounted at an angle that transmits the 532 nm green laser or 577 nm yellow laser beam and reflects the 635 nm aiming beam.

It is another aspect of the present invention, wherein the laser beam from the fiber optic cable passes through the focusing lens assembly to get a required spot size of 100 microns to 300 microns.

It is another aspect of the present invention, wherein the folding mirror is mounted in an angle of 45 degree with a tolerance level of + or - 2 degrees along the laser optical axis.

It is another aspect of the present invention, wherein the dual wavelength laser delivery system converges and focuses the laser wavelength of 532 nm and/or 577 nm laser beam in-line with the 635 nm aiming beam to create a circular pattern to accomplish a pre-measured Capsulorhexis with a precise circular cut of the capsule in the human eye with a precise diameter of 3 mm to 6 mm in steps of 0.5 mm.

It is another aspect of the present invention, wherein the laser unit comprises a foot switch to trigger the 532 nm and/or 577 nm laser beam.

### BRIEF DESCRIPTION OF THE DRAWINGS

Figure 1: illustrates dual wavelength laser delivery system according to the present invention.
Figure 2: illustrates block diagram of dual wavelength laser delivery system according to the present invention.
Figure 3: illustrates laser unit of the system according to the present invention.
Figure 4: illustrates dual wavelength laser delivery system comprising an existing ophthalmic operating microscope according to the present invention.
Figure 5: illustrates delivery system and laser beams according to the present invention.
Figure 6: illustrates the aiming beam RED laser spot and circular diameter of 5.5 mm.
Figure 7: illustrates the proof of conformity in lab trials of Capsulorhexis in animal eye samples for 5.5 mm and 6 mm diameter.
Figurer 8: illustrates the proof of Capsulorhexis trials conducted on animal eyes.

### DETAILED DESCRIPTION OF THE INVENTION WITH REFERENCE TO THE ACCOMPANYING FIGURES

The present invention as herein described relates to a dual (532 and 577) nm laser delivery system comprising an existing ophthalmic operating microscope for creation of circular pattern to accomplish a pre-measured diameter size of capsulorhexis.

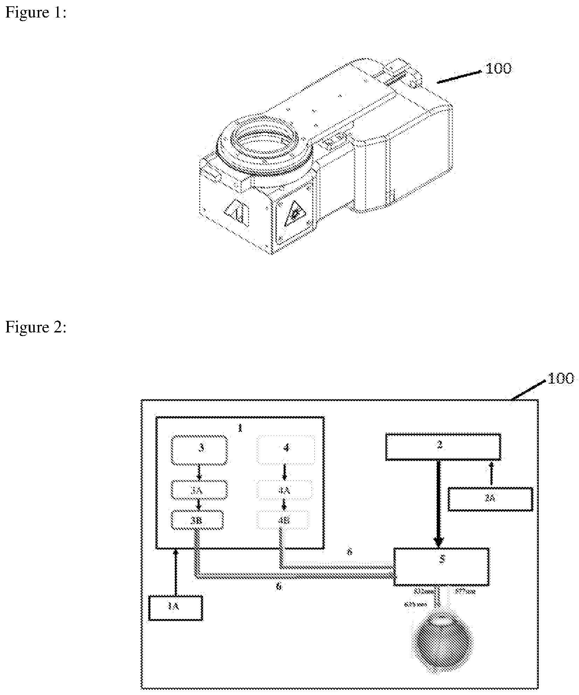

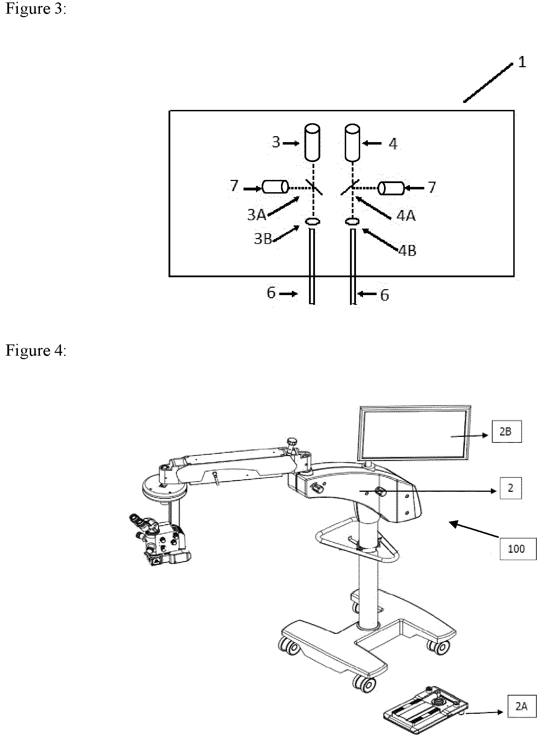

Referring to Figures 1 to 3, the dual wavelength laser delivery system 100 comprises of a dual wavelength laser unit 1; a delivery system 5; and an ophthalmic operating microscope 2. The laser unit 1 comprises of a green laser module 532 nm 3 and a yellow laser module 577 nm 4 along with 635 nm aiming beam 7 (as shown in Figure 3) within both the laser modules 3,4 to create a circular pattern with a precise circular cut of the capsule in the human eye. The 635 nm aiming beam 7 is used to locate and guide the user to aim the 535 nm and/or 577 nm laser beam on the human eye capsule before the Capsulorhexis. The green laser module 3 comprises of a low pass filter 3A and a focusing lens 3B. The yellow laser module 4 comprises of a low pass filter 4A and a focusing lens 4B. The low pass filters 3A, 4A are mounted at an angle that transmits the 532 nm green laser 3 or 577 nm yellow laser beam 4 and reflects the 635 nm aiming beam 7. The laser modules 3,4 are connected to the delivery system 5 through an interchangeable single fiber optic cable 6. The laser unit 1 includes a foot switch 1A to trigger the laser beam.

Referring to Figure 4, the dual wavelength laser delivery system 100 comprises the operating microscope's 2 optical head . Said laser delivery system 100 converges and focuses laser wavelength of 532 nm and/or 577 nm laser beam in-line with 635 nm aiming beam to create a circular pattern in order to accomplish a pre-measured Capsulorhexis with a precise circular cut of the capsule in the human eye. The ophthalmic microscope 2 includes a foot switch 2A for focus adjustment and magnification changes of the operating microscope. The ophthalmic microscope 2 includes a display unit 2B to display the live image of Capsulorhexis procedure.

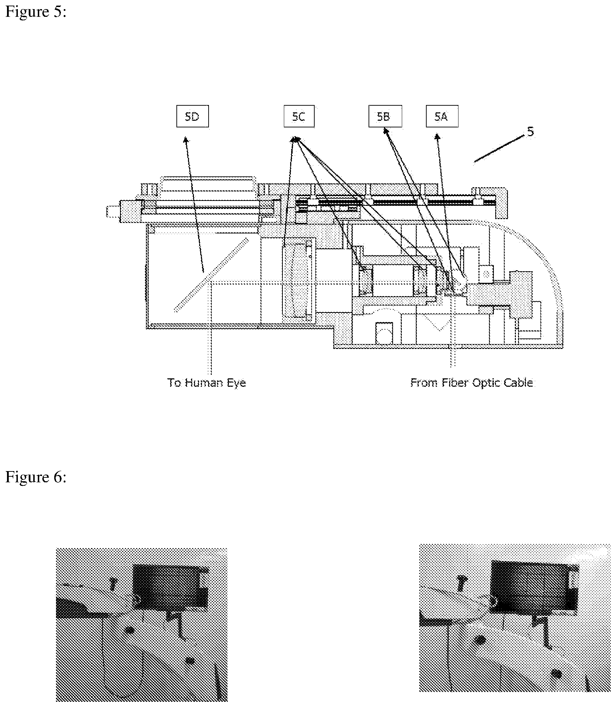

Referring to Figure 5, the delivery system 5 comprises of a right-angle prism 5A and an X and Y Galvo assembly 5B to generate required laser deflection and a circular laser pattern. The laser beam from the fiber optic cable 6 passes through a focusing lens assembly 5C to get a required spot size of 100 microns to 300 microns. The laser beam is focused on the human eye using a folding mirror 5D. The focusing lens assembly 5C includes four focusing lenses. The folding mirror 5D is at along the laser optical axis that deflects the laser beam 90 degrees into the human eye capsule to perform the Capsulorhexis. In an aspect, the folding mirror 5D is mounted on an optics assembly in an angle of 45 degree with a tolerance level of + or - 2 degrees.

The delivery system 5 converges and focuses laser wavelength of 532 nm and 577nm laser beam to create a circular pattern in order to accomplish a pre-measured Capsulorhexis with a precise circular cut of the capsule in the human eye from diameter 3 mm to 6 mm in steps of 0.5 mm. Figure 6 shows the aiming beam RED laser spot and circular diameter of 5.5 mm.

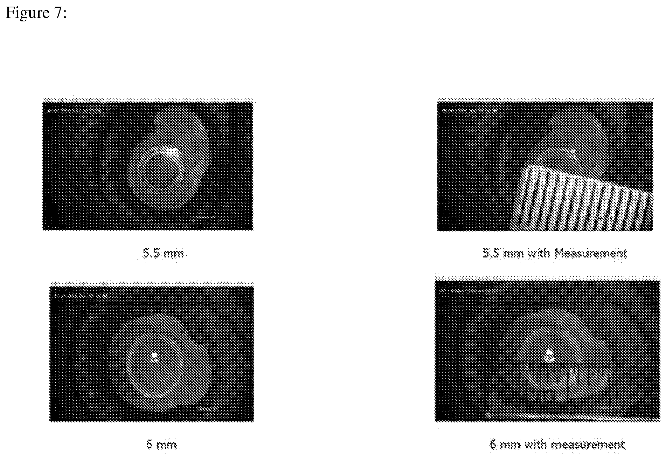

A number of trials of capsulorhexis in animal eyes have been conducted using the dual (532 and 577) nm laser delivery system and have achieved the capsulorhexis in the diameter of 5.5mm and 6mm. Figure 7 illustrates the proof of conformity in lab trials of Capsulorhexis in animal eye samples for 5.5 mm and 6 mm diameter.

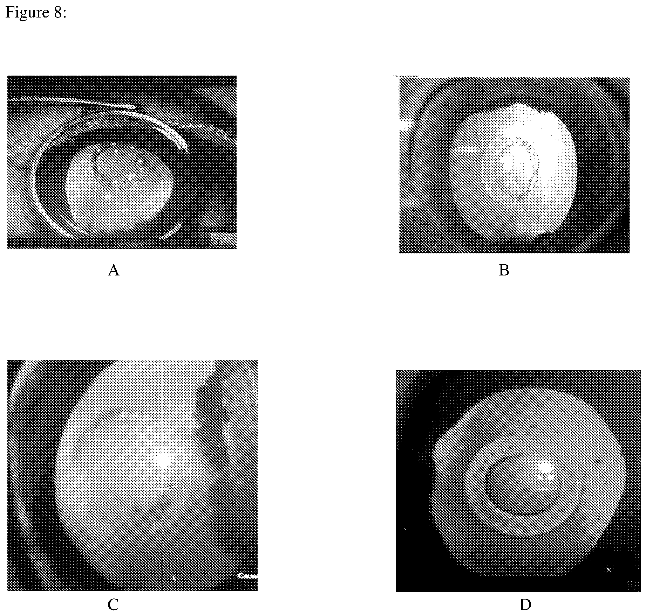

Animal eye lab trials were being conducted from 2020, and have completed 17 trials so far. This process was evolved using different laser powers, laser ON time duration. Standard ophthalmic viscoelastic solution was used to protect the Corneal endothelium cells during the laser ablation procedure and for better capsule identification ophthalmic blue dye solution is used for staining the capsule. Also different concentration of the Ophthalmic blue dye solution was used to observe the absorption level of the two different laser wavelengths (532/577) nm on the capsule tissue under study. Figure 8A shows the first trial; 8B shows the 10^{th} trial; 8C shows the 12^{th} trial and 8D shows the 15^{th} trial.

The dual wavelength laser delivery system 100 comprises an existing ophthalmic microscope ; said system is mounted along the optical axis to suit the requirement, so that it does not obscure the Surgeon's view while operating. The dual wavelength laser delivery system 100 comprising an existing ophthalmic microscope saves Surgeon time since Patient is not required to be shifted from one Surgical equipment to the other Surgical equipment after the Capsulorhexis is completed, produce accuracy and repeatability in creation of a precise diameter of the capsulorhexis from diameter 3 mm to 6 mm in steps of 0.5 mm. Advantageously, the dual wavelength laser delivery system 100 along with the aiming beam provides flexibility and compatibility to the ophthalmic Surgeons.

### Working:

For cataract surgery in a human eye, the crystalline lens is replaced with an Intraocular Lens (IOL). The crystalline lens is enclosed in a capsule on front and back side. In conventional cataract surgery, the front portion (anterior) of capsule is circularly removed by 26g needle or capsular forceps and IOL is placed. The present invention is to a system 100 which performs laser assisted procedure to speed up the Cataract surgery of removing the anterior of capsule from diameter 3 mm to 6 mm. In an aspect, anyone of the two wavelengths 532 nm 3 or 577 nm 4 at a given point of time selected by the user is used along with guiding/aiming beam 7 of wavelength 635 nm. The 532 nm or 577 nm is generated in the laser unit 1. The 532 nm and 577 nm laser along with aiming beam is coupled by focusing lens while leaving the laser unit. The 532 nm and 577 nm laser beams will enter one end of the fiber optic cable 6 which works in total internal reflection principle without loss and leave the other end. The other end of the fiber optic cable 6 is connected to the delivery system 5 which is mounted in the operating microscope 2. These lasers beams leaving the other end of the fiber optic cable 6 passes through the right angle prism 5A, gets deflected by the X and Y Galvo assembly 5B, enters the focusing lens assembly 5C. These lasers beams after passing through the focusing lens assembly 5C is reflected/folded by the folding mirror 5D and focused on the human eye capsule. By choosing the required laser 532 nm and 577 nm and diameter and proper focusing of the microscope, the Capsulorhexis is performed using the foot switch 1A.

## Claims

1. A dual wavelength laser delivery system (100), comprising:
a dual wavelength laser unit (1);
a delivery system (5); and
an ophthalmic operating microscope (2),
wherein the dual wavelength laser unit (1) comprises of a green laser module 532 nm (3) and a yellow laser module 577 nm (4) along with an aiming beam (7) 635 nm *]* within both the laser modules (3,4) to create a circular pattern with a precise circular cut of the capsule in a human eye,
wherein the aiming beam 635 nm (7) is used to locate and guide user to aim the 535 nm and/or 577 nm laser beam,
wherein each of the laser modules (3, 4) comprises of a low pass filter (3A,4A) and a focusing lens (3B, 4B) respectively,
wherein the green and yellow laser modules (3,4) are connected to the delivery system (5) through an interchangeable single fiber optic cable (6),
wherein the delivery system (5) comprises of a right-angle prism (5A); a X and Y Galvo assembly (5B) a focusing lens assembly (5C) and a folding mirror (5D)
wherein the wherein the laser beams generated in the green laser module 532 nm (3) and yellow laser module 577 nm (4) from the laser unit (1) pass through the fiber optic cable (6)gets reflected using the right angle prism (5A), gets deflected by the X and Y Galvo assembly (5B), enters the focusing lens assembly (5C) and gets reflected by a folding mirror (5D) and focused on the human eye capsule at 90 degrees, and
wherein said laser delivery system (100) converges and focuses the laser wavelength of 532 nm and/or 577 nm laser beam in-line with the 635 nm aiming beam (7) to create a circular pattern to accomplish a pre-measured Capsulorhexis with a precise circular cut of the capsule
in the human eye.

2. The dual wavelength laser delivery system (100) as claimed in claim 1, wherein the low pass filters (3A,4A) are mounted at an angle that transmits the 532 nm green laser (3) or 577 nm yellow laser beam (4) and reflects the 635 nm aiming beam (7).

3. **The dual** wavelength laser delivery system (100) as claimed in claim 1, wherein the laser beam from the fiber optic cable (6) passes through the focusing lens assembly (5C) to get a required spot size of 100 microns to 300 microns.

4. The dual wavelength laser delivery system (100) as claimed in claim 1, wherein the folding mirror (5D) is mounted in an angle of 45 degree with a tolerance level of + or - 2 degrees along the laser optical axis.

5. The dual wavelength laser delivery system (100) as claimed in claim 1 converges and focuses the laser wavelength of 532 nm and/or 577 nm laser beam in-line with the 635 nm aiming beam (7) to create a circular pattern to accomplish a pre-measured Capsulorhexis with a precise circular cut of the capsule in the human eye with a precise diameter of 3 mm to 6 mm in steps of 0.5 mm.

6. The dual wavelength laser delivery system (100) as claimed in claim 1, wherein the laser unit (1) comprises a foot switch (1A) to trigger the 532 nm and/or 577 nm laser beam.

## Patentansprüche

1. Ein Laserzuführungssystem mit zwei Wellenlängen (100), umfassend:
eine Lasereinheit mit zwei Wellenlängen (1);
ein Zuführungssystem (5); und
ein ophthalmisches Operationsmikroskop (2),
wobei die Lasereinheit mit zwei Wellenlängen (1) ein grünes Lasermodul 532 nm (3) und ein gelbes Lasermodul 577 nm (4) zusammen mit einem Zielstrahl (7) 635 nm ] innerhalb beider Lasermodule (3,4) zur Erzeugung eines kreisförmigen Musters mit einem präzisen kreisförmigen Schnitt der Kapsel in einem menschlichen Auge umfasst,
wobei der Zielstrahl 635 nm (7) zum Lokalisieren und Führen des Benutzers beim Ausrichten des 535 nm und/oder 577 nm Laserstrahls verwendet wird,
wobei jedes der Lasermodule (3, 4) einen Tiefpassfilter (3A,4A) beziehungsweise eine Fokussierlinse (3B, 4B) umfasst,
wobei die grünen und gelben Lasermodule (3,4) über ein austauschbares einzelnes Glasfaserkabel (6) mit dem Zuführungssystem (5) verbunden sind,
wobei das Zuführungssystem (5) ein rechtwinkliges Prisma (5A); eine X- und Y-Galvo-
Anordnung (5B) eine Fokussierlinsenanordnung (5C) und einen Umlenkspiegel (5D) umfasst
wobei wobei die in dem grünen Lasermodul 532 nm (3) und dem gelben Lasermodul 577 nm (4) von der Lasereinheit (1) erzeugten Laserstrahlen durch das Glasfaserkabel (6) verlaufen mittels des rechtwinkligen Prismas (5A) reflektiert werden, durch die X- und Y-Galvoanordnung (5B) abgelenkt werden, in die Fokussierlinsenanordnung (5C) eintreten und durch einen Umlenkspiegel (5D) reflektiert und bei 90 Grad auf die Kapsel des menschlichen Auges fokussiert werden, und
wobei das genannte Laserzuführungssystem (100) die Laserwellenlänge des Laserstrahls von 532 nm und/oder 577 nm in einer Linie mit dem 635 nm Zielstrahl (7) zur Erzeugung eines kreisförmigen Musters zur Durchführung einer vorab bemessenen Kapsulorhexis mit einem präzisen kreisförmigen Schnitt der Kapsel in dem menschlichen Auge bündelt und fokussiert.

2. Das Laserzuführungssystem mit zwei Wellenlängen (100) nach Anspruch 1, wobei die Tiefpassfilter (3A,4A) unter einem Winkel zum Durchlassen des grünen 532 nm Lasers (3) oder des gelben 577 nm Laserstrahls (4) und zum Reflektieren des 635 nm Zielstrahls (7) montiert sind.

3. Das Laserzuführungssystem mit zwei Wellenlängen (100) nach Anspruch 1, wobei der Laserstrahl von dem Glasfaserkabel (6) zum Erhalten einer erforderlichen Fleckgröße von 100 Mikrometern bis 300 Mikrometern durch die Fokussierlinsenanordnung (5C) verläuft.

4. Das Laserzuführungssystem mit zwei Wellenlängen (100) nach Anspruch 1, wobei der Umlenkspiegel (5D) unter einem Winkel von 45 Grad mit einem Toleranzniveau von + oder - 2 Grad entlang der optischen Laserachse montiert ist.

5. Das Laserzuführungssystem mit zwei Wellenlängen (100) nach Anspruch 1 bündelt und fokussiert die Laserwellenlänge des Laserstrahls von 532 nm und/oder 577 nm in einer Linie mit dem 635 nm Zielstrahl (7) zur Erzeugung eines kreisförmigen Musters zur Durchführung einer vorab bemessenen Kapsulorhexis mit einem präzisen kreisförmigen Schnitt der Kapsel in dem menschlichen Auge mit einem präzisen Durchmesser von 3 mm bis 6 mm in Schritten von 0.5 mm.

6. Das Laserzuführungssystem mit zwei Wellenlängen (100) nach Anspruch 1, wobei die Lasereinheit (1) einen Fußschalter (1A) zum Auslösen des 532 nm und/oder 577 nm Laserstrahls umfasst.

## Revendications

1. Un système d'acheminement laser à double longueur d'onde (100), comprenant:
une unité laser à double longueur d'onde (1);
un système d'acheminement (5); et
un microscope opératoire ophtalmique (2),
dans lequel l'unité laser à double longueur d'onde (1) comprend un module laser vert 532 nm (3) et un module laser jaune 577 nm (4) conjointement avec un faisceau de visée (7) 635 nm ] à l'intérieur des deux modules laser (3,4) pour créer un motif circulaire avec une découpe circulaire précise de la capsule dans un œil humain,
dans lequel le faisceau de visée 635 nm (7) est utilisé pour localiser et guider l'utilisateur afin de viser le faisceau laser de 535 nm et/ou 577 nm,
dans lequel chacun des modules laser (3, 4) comprend respectivement un filtre passe-bas (3A,4A) et une lentille de focalisation (3B, 4B),
dans lequel les modules laser vert et jaune (3,4) sont raccordés au système d'acheminement (5) au moyen d'un câble à fibre optique unique interchangeable (6),
dans lequel le système d'acheminement (5) comprend un prisme à angle droit (5A); un ensemble Galvo X et Y (5B) un ensemble de lentille de focalisation (5C) et un miroir de repliement (5D)
dans lequel dans lequel les faisceaux laser générés dans le module laser vert 532 nm (3) et le module laser jaune 577 nm (4) par l'unité laser (1) traversent le câble à fibre optique (6) sont réfléchis à l'aide du prisme à angle droit (5A), sont déviés par l'ensemble Galvo X et Y (5B), entrent dans l'ensemble de lentille de focalisation (5C) et sont réfléchis par un miroir de repliement (5D) et focalisés sur la capsule de l'œil humain à 90 degrés, et
dans lequel ledit système d'acheminement laser (100) fait converger et focalise la longueur d'onde laser du faisceau laser de 532 nm et/ou 577 nm en ligne avec le faisceau de visée 635 nm (7) pour créer un motif circulaire afin d'effectuer une Capsulorhexis pré-mesurée avec une découpe circulaire précise de la capsule dans l'œil humain.

2. Le système d'acheminement laser à double longueur d'onde (100) selon la revendication 1, dans lequel les filtres passe-bas (3A,4A) sont montés sous un angle qui transmet le laser vert 532 nm (3) ou le faisceau laser jaune 577 nm (4) et réfléchit le faisceau de visée 635 nm (7).

3. Le système d'acheminement laser à double longueur d'onde (100) selon la revendication 1, dans lequel le faisceau laser provenant du câble à fibre optique (6) traverse l'ensemble de lentille de focalisation (5C) pour obtenir une dimension de tache requise de 100 micromètres à 300 micromètres.

4. Le système d'acheminement laser à double longueur d'onde (100) selon la revendication 1, dans lequel le miroir de repliement (5D) est monté sous un angle de 45 degrés avec un niveau de tolérance de + ou - 2 degrés le long de l'axe optique du laser.

5. Le système d'acheminement laser à double longueur d'onde (100) selon la revendication 1 fait converger et focalise la longueur d'onde laser du faisceau laser de 532 nm et/ou 577 nm en ligne avec le faisceau de visée 635 nm (7) pour créer un motif circulaire afin d'effectuer une Capsulorhexis pré-mesurée avec une découpe circulaire précise de la capsule dans l'œil humain ayant un diamètre précis de 3 mm à 6 mm par pas de 0.5 mm.

6. Le système d'acheminement laser à double longueur d'onde (100) selon la revendication 1, dans lequel l'unité laser (1) comprend un interrupteur à pédale (1A) pour déclencher le faisceau laser de 532 nm et/ou 577 nm.
